# EUROPÄISCHE PATENTANMELDUNG

(11) **EP 4 357 325 A1**
(43) Veröffentlichungstag der Anmeldung: **24.04.2024**
(21) Anmeldenummer: 22202896.1
(22) Anmeldetag: 21.10.2022
(51) Int. Cl.: C07B 59/00, C07C 29/16, C07C 33/22, C07C 33/20, C07C 33/02

(54) **VERFAHREN ZUR HERSTELLUNG EINES MEHRFACH DEUTERIERTEN ALKOHOLS MITTELS KUPFER-KATALYSATORS**

(71) Anmelder: Evonik Operations GmbH, 45128 Essen (DE)
(72) Erfinder: WU, Xiao-Feng, 18059 Rostock (DE); GENG, Hui-Qing, 18059 Rostock (DE); BÖRNER, Armin, 18059 Rostock (DE); FRANKE, Robert, 45772 Marl (DE)
(74) Vertreter: Evonik Patent Association

(57) **Zusammenfassung**

Verfahren zur Herstellung eines mehrfach deuterierten Alkohols mittels Kupfer-Katalysators.

## Beschreibung

Die Erfindung betrifft ein Verfahren zur Herstellung eines mehrfach deuterierten Alkohols mittels Kupfer-Katalysators.

Die technische Aufgabe, welche der vorliegenden Erfindung zugrunde lag, ist die Bereitstellung eines Verfahrens zur Herstellung eines mehrfach deuterierten Alkohols.

Diese Aufgabe wird gelöst durch ein Verfahren nach Anspruch 1.

Verfahren umfassend die Verfahrensschritte:
a) Vorlegen eines Olefins;
b) Zugabe eines Liganden gemäß Formel (I): 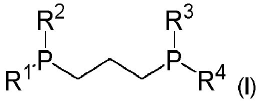 wobei R¹, R², R³, R⁴ ausgewählt sind aus: -(C₁-C₁₂)-Alkyl, -(C₆-C₁₀)-Aryl;
c) Zugabe einer Verbindung, welche Cu umfasst;
d) Zuführen von D₂;
f) Zuführen von CO;
g) Erwärmen des Reaktionsgemisches aus a) bis f), wobei das Olefin zu einem deuterierten Alkohol umgesetzt wird.

In einer Variante des Verfahrens stehen R¹ und R² für -(C₆-C₁₀)-Aryl.

In einer Variante des Verfahrens stehen R¹ und R² für -Ph.

In einer Variante des Verfahrens stehen R¹ und R³ für -(C₆-C₁₀)-Aryl.

In einer Variante des Verfahrens stehen R¹ und R³ für -Ph.

In einer Variante des Verfahrens weist der Ligand die Struktur (1) auf:

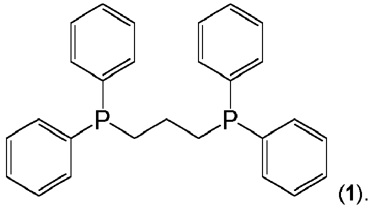

In einer Variante des Verfahrens ist die Verbindung, welche Cu umfasst, ausgewählt aus: CuCl, [Cu(OTf)], Cul, CuBr, Cu(OAc)₂.

In einer Variante des Verfahrens ist die Verbindung, welche Cu umfasst, CuCl.

In einer Variante des Verfahrens erfolgt das Zuführen von D₂ in Verfahrensschritt d) mit einem Druck in dem Bereich von 0,5 bis 5 MPa (5 bis 50 bar).

In einer Variante des Verfahrens erfolgt das Zuführen von CO in Verfahrensschritt f) mit einem Druck in dem Bereich von 0,5 bis 5 MPa (5 bis 50 bar).

In einer Variante des Verfahrens erfolgt das Erwärmen des Reaktionsgemisches in Verfahrensschritt g) auf eine Temperatur in dem Bereich von 80 °C bis 140 °C.

In einer Variante des Verfahrens umfasst dieses den zusätzlichen Verfahrensschritt f'): f') Zugabe eines Lösungsmittels.

In einer Variante des Verfahrens ist das Lösungsmittel ausgewählt aus: CH₃CN, DMSO, DMF, *^{tert}*BuOH, Toluol, Cyclohexan.

In einer Variante des Verfahrens ist das Lösungsmittel Cyclohexan.

Im Folgenden soll die Erfindung anhand eines Ausführungsbeispiels näher erläutert werden.

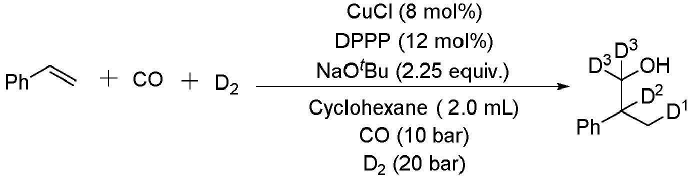

Ein getrocknetes 4-mL-Schraubdeckelgefäß, das mit einem Septum und einem Rührstab ausgestattet war, wurde mit CuCI (8 mol%), 1,3-Bis(diphenylphosphino)propan (DPPP) (12 mol%), NaO^{t}Bu (2.25 equi.) und 30 mg Molekularsieb (0.3 nm = 3 Å) befüllt. Das Fläschchen wurde versiegelt, mit einer kleinen Kanüle an die Atmosphäre angeschlossen, evakuiert und mit Argon aufgefüllt. Cyclohexan (2,0 mL) wurden per Spritze unter Argon-Gegenstrom zugegeben. Zu dieser Suspension wurde Styrol (0,4 mmol) über eine Hamilton^{®}-Spritze zugegeben. Das Fläschchen wurde auf eine Metallplatte gestellt und in einen 300 mL Autoklaven (Serie 4560 von Parr instrument company^{®}) überführt. Der Autoklav wurde einmal mit Stickstoff (10 bar) und drei Mal mit CO (10 bar) gespült. Anschließend wurde der Autoklav mit CO (10 bar) und mit D₂ (20 bar) befüllt. Der Autoklav wurde dann in einen Aluminiumblock auf einen Magnetrührer gestellt. Das Reaktionsgemisch wurde 24 h lang bei 100 °C gerührt (600 U/min). Anschließend wurde es auf Raumtemperatur abgekühlt und der Druck vorsichtig abgelassen. Eine NH₄Cl-Lösung (2 mL) wurde zugegeben und 10 Minuten gerührt. Die Mischung wurde mit Essigsäureethylester (10 mL) extrahiert. Die vereinigten organischen Phasen wurden über Na₂SO₄ getrocknet und im Vakuum konzentriert. Der Rückstand wurde durch Säulenchromatographie (Pentan:Essigsäureethylester = 5:1) gereinigt. Der Alkohol wurde als farbloses Öl erhalten (32 mg, 58 %).

NMR (2-Phenylpropan-1,1,2,3-*d₄*-1-ol)

¹H-NMR (300 MHz, CDCl₃): δ 7.41 - 7.33 (m, 2H), 7.30 - 7.22 (m, 3H), 1.54 (s, 1H), 1.30 - 1.27 (m, 2H).

¹³C-NMR (75 MHz, CDCl₃): δ 143.7, 128.7, 127.5, 126.7, 17.2 (t, *J* = 20.3 Hz).

Wie das Ausführungsbeispiel zeigt, wird die Aufgabe durch das erfindungsgemäße Verfahren gelöst.

## Patentansprüche

1. Verfahren umfassend die Verfahrensschritte:
a) Vorlegen eines Olefins;
b) Zugabe eines Liganden gemäß Formel (I): 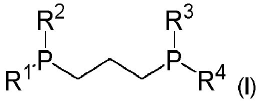 wobei R¹, R², R³, R⁴ ausgewählt sind aus: -(C₁-C₁₂)-Alkyl, -(C₆-C₁₀)-Aryl;
c) Zugabe einer Verbindung, welche Cu umfasst;
d) Zuführen von D₂;
f) Zuführen von CO;
g) Erwärmen des Reaktionsgemisches aus a) bis f), wobei das Olefin zu einem deuterierten Alkohol umgesetzt wird.

2. Verfahren nach Anspruch 1,
wobei R¹ und R² für -(C₆-C₁₀)-Aryl stehen.

3. Verfahren nach einem der Ansprüche 1 oder 2,
wobei R¹ und R² für -Ph stehen.

4. Verfahren nach einem der Ansprüche 1 bis 3,
wobei R¹ und R³ für -(C₆-C₁₀)-Aryl stehen.

5. Verfahren nach einem der Ansprüche 1 bis 4,
wobei R¹ und R³ für -Ph stehen.

6. Verfahren nach einem der Ansprüche 1 bis 5,
wobei der Ligand die Struktur (1) aufweist:

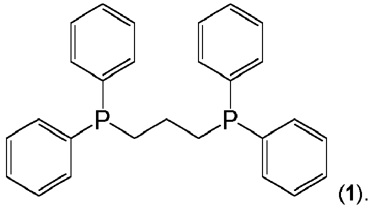

7. Verfahren nach einem der Ansprüche 1 bis 6,
wobei die Verbindung, welche Cu umfasst, ausgewählt ist aus: CuCI, [Cu(OTf)], Cul, CuBr, Cu(OAC)₂.

8. Verfahren nach einem der Ansprüche 1 bis 7,
wobei die Verbindung, welche Cu umfasst, CuCI ist.

9. Verfahren nach einem der Ansprüche 1 bis 8,
wobei das Zuführen von D₂ in Verfahrensschritt d) mit einem Druck in dem Bereich von 0,5 bis 5 MPa (5 bis 50 bar) erfolgt.

10. Verfahren nach einem der Ansprüche 1 bis 9,
wobei das Zuführen von CO in Verfahrensschritt f) mit einem Druck in dem Bereich von 0,5 bis 5 MPa (5 bis 50 bar) erfolgt.

11. Verfahren nach einem der Ansprüche 1 bis 10,
wobei das Erwärmen des Reaktionsgemisches in Verfahrensschritt g) auf eine Temperatur in dem Bereich von 80 °C bis 140 °C erfolgt.

12. Verfahren nach einem der Ansprüche 1 bis 11,
umfassend den zusätzlichen Verfahrensschritt f'):
f') Zugabe eines Lösungsmittels.

13. Verfahren nach Anspruch 12,
wobei das Lösungsmittel ausgewählt ist aus: CH₃CN, DMSO, DMF, *^{tert}*BuOH, Toluol, Cyclohexan.

14. Verfahren nach einem der Ansprüche 12 oder 13,
wobei das Lösungsmittel Cyclohexan ist.
